# EUROPÄISCHE PATENTSCHRIFT

(11) **EP 0 809 623 B1**
(45) Veröffentlichungstag und Bekanntmachung des Hinweises auf die Patenterteilung: **19.01.2000**
(21) Anmeldenummer: 96904795.0
(22) Anmeldetag: 15.02.1996
(51) Int. Cl.: C07C 69/593, C07C 69/34, C11D 1/74, A61K 7/46, A61K 31/23, A61K 7/50, A01N 37/06, C07C 67/08, A23L 1/03

(54) **ALK(EN)YLDICARBONSÄUREBISESTER, DEREN VERWENDUNG SOWIE VERFAHREN ZU DEREN HERSTELLUNG**
ALK(EN)YL DICARBOXYLIC ACID BISESTERS, THEIR USE AND METHOD FOR PREPARING THEM
BIS-ESTERS D'ACIDE ALC(EN)YL DICARBOXYLIQUE, LEUR UTILISATION ET LEUR PROCEDE DE PREPARATION

(30) Priorität: 15.02.1995 DE 19505100
(43) Veröffentlichungstag der Anmeldung: 03.12.1997
(73) Patentinhaber: BASF AKTIENGESELLSCHAFT, 67056 Ludwigshafen (DE)
(72) Erfinder: DRALLE-VOSS, Gabriele, D-64297 Darmstadt (DE); OETTER, Günter, D-67227 Frankenthal (DE); WEKEL, Hans-Ulrich, D-67158 Ellerstadt (DE); OPPENLÄNDER, Knut, D-67061 Ludwigshafen (DE)
(74) Vertreter: Isenbruck, Günter, Dr.
(86) Internationale Anmeldenummer: EP9600657
(87) Internationale Veröffentlichungsnummer: WO9625384

(56) Entgegenhaltungen:
- WO-A-94/00508
- DD-A- 40 624
- DE-A- 4 238 032
- US-A- 2 650 211

## Beschreibung

Die vorliegende Erfindung betrifft oligomere und polymere Bisester aus Alkyl- oder Alkenyldicarbonsäurederivaten und Polyalkoholen sowie deren Verwendung als Solubilisatoren, Emulgatoren und/oder waschaktive Substanzen. Insbesondere werden die Bisester vorteilhaft in kosmetischen Zusammensetzungen, Wasch- und Reinigungsmitteln, pharmazeutischen Zusammensetzungen, Lebensmitteln und Pflanzenschutzmitteln verwendet. Weiterhin betrifft die Erfindung ein Verfahren zur Herstellung dieser Bisester.

Auf dem Tensid- und Emulgatoren-Sektor sind in den letzten Jahren zunehmend nichtionische oberflächenaktive Substanzen in den Vordergrund getreten, die gut biologisch abbaubar sind, eine geringe Toxizität aufweisen und somit eine gute Umweltverträglichkeit besitzen.

Eine wichtige Gruppe solcher nichtionischen Verbindungen sind Ester aus Fettsäuren und Polyalkoholen, wie Saccharoseester, Sorbitanester und Glycerin- und Polyglycerinfettsäureester. Bei diesen Verbindungen ist eine Polyalkoholgruppe mit einem hydrophoben Fettsäurerest verestert.

Die meisten Produkte der genannten Verbindungsklassen sind zu unpolar, um in wäßrigen Formulierungen als Tensid eingesetzt zu werden. Die hydrophileren Produkte dieses Verbindungstyps erweisen sich in einigen Eigenschaften wie dem Schaum- und Netzvermögen sowie der Grenz- und Oberflächenspannung als noch verbesserungsbedürftig.

Die JP 051 25 014 offenbart Monoester von Alkenylcarbonsäureanhydriden mit hydrophilen Verbindungen, wie Polyglycerin, und deren Einsatz als Tenside.

Die WO 94/00508 offenbart Tenside, bei denen eine Polyethylenglykolkette an einen Alk(en)ylbernsteinsäurerest gebunden ist. Die beschriebenen Polyethylenglykolbisester besitzen mindestens 13 Kohlenstoffatome.

Die DE 42 38 032 offenbart Bisester von Alk(en)ylbernsteinsäurederivaten mit Polyethylenglykolen und deren Verwendung als Hautkonditionierungsmittel.

Die EP 01 07 199 und die BE 898 441 offenbaren Polyoxyalkylenmonoester von Alk(en)ylbernsteinsäurederivaten sowie verschiedene Einsatzzwecke dieser Verbindungen.

Von Nachteil sind bei diesen Produkten insbesondere die mangelnde Hartwasserbeständigkeit und die zu hohen Grenzflächenspannungen und damit die zu geringe Solubilisier- und Emulgierwirkung.

Die J 6 00 18 584 offenbart Alkenylbernsteinsäurebisglycerinester zur Verwendung als Korrosionsinhibitoren in öllöslichem Medium.

In der SU 457 754 und in der DL 163 348 werden Alkenylbernsteinsäurepolyglycerinbisester in Elektrolyten für Versilberungen bzw. für Zinnbäder eingesetzt.

Die J 5 70 65 793, die SU 432 172, die US 4 234 435 und die US 4 159 958 offenbaren Anwendungen von Alkenylbernsteinsäurepolyglycerinmonoestern oder -bisestern in Schmierölen und Kühlschmiermitteln.

Die DD 40 624 offenbart ein Verfahren zur Herstellung von Dicarbonsäurepolyglycerinbisestern und erwähnt die Netzmitteleigenschaften dieser Verbindungen.

DD-A-40624 betrifft hautkonditionierende Bernsteinsäure-Derivate, die lipidanaloge Eigenschaften aufweisen, die sie als hautfeuchtigkeitsregulierende Komponente in kosmetischen oder therapeutischen Zubereitungen zur Behandlung der Haut geeignet machen. Die offenbarten Verbindungen weisen die Sequenz Diol-Bernsteinsäure-Diol auf, wobei eine der endständigen Hydroxylgruppen der Diol-Komponenten mit einem C₆ bis 22- Alkylrest verethert ist. Ebenfalls offenbart werden Verbindungen der Sequenz Diol-Bernsteinsäure-Diol-Bernsteinsäure-Diol, wobei beide endständigen Hydroxylgruppen der endständigen Diole mit C₆ bis 22- Resten verethert sind.

Der vorliegenden Erfindung lag daher die Aufgabe zugrunde, Verbindungen bereitzustellen, die als waschaktive Substanzen, Solubilisatoren und Emulgatoren brauchbar sind und die die oben beschriebenen Nachteile nicht aufweisen. Die erfindungsgemäßen Verbindungen sollten ihre vorteilhaften Wirkungen insbesondere in wäßrigen bzw. in wäßrig/ethanolischen Systemen entfalten können, in denen darin schwer- oder unlösliche Substanzen solubilisiert bzw. emulgiert werden sollen oder aus denen solche Substanzen mit Hilfe der Waschaktivität der erfindungsgemäßen Verbindungen entfernt werden sollen.

Insbesondere sollen die erfindungsgemäßen Verbindungen in Wasch- und Reinigungsmitteln sowie in kosmetischen Zusammensetzungen brauchbar sein. Weiterhin sollen die Verbindungen physiologisch bzw. pflanzenphysiologisch inert sein, um sie in Pflanzenschutzmitteln, pharmazeutischen Zusammensetzungen oder in diätetischen und nicht-diätetischen Lebensmitteln einsetzen zu können. Von Vorteil wären bei den beiden zuletzt genannten Anwendungsbereichen zudem ein geringer Eigengeruch sowie ein neutraler Eigengeschmack.

Die Aufgaben werden durch die im folgenden beschriebenen Oligo- und Poly-Bisester von Malonsäure-, Bernsteinsäure- und Glutarsäurederivaten mit Polyalkoholen sowie deren Herstellung und Verwendung gelöst. Die Bisester haben nicht nur sehr gute waschaktive, solubilisatorische und emulgatorische Eigenschaften, sondern auch gegenüber den beschriebenen Monoestern eine verbesserte Stabilität gegen die Wasserhärte und niedrigere Grenzflächenspannungen gegen verschiedene hydrophobe Stoffe, wie beispielsweise Öle. Außerdem besitzen sie einen geringen Eigengeruch und einen neutralen Eigengeschmack.

Einen erfindungsgemäßen Gegenstand stellt somit eine Verbindung der Formel I dar

(H⊙)_{a-b}A(⊙X)_{b} (I)

worin
- A: den Rest eines monomeren Polyalkohols oder eines oligomeren Polyalkohols, der aus bis zu 20 über Etherbindungen miteinander verknüpften Monomeren besteht, bedeutet, wobei der monomere Polyalkohol bzw. das Monomer des oligomeren Polyalkohols ein Alkohol ist, der wenigstens 3 Kohlenstoffatome sowie a OH-Gruppen aufweist;
- a: ≥ 2 ist;
- ⊙: jeweils ein Sauerstoffatom einer der OH-Gruppen des Restes A bedeutet;
- X: einen Rest der Formel II 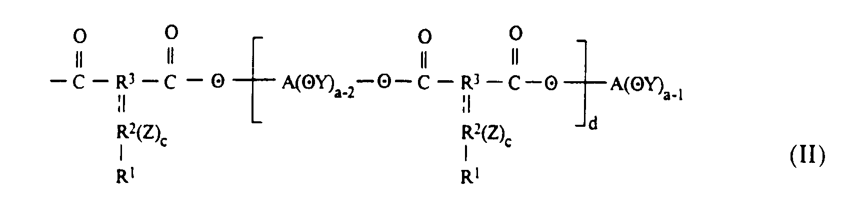
bedeutet;
worin
- A, ⊙ und a: wie oben definiert sind;
- Y: ein Wasserstoffatom, oder einen Rest X, der wie oben definiert ist, bedeutet;
- R¹: einen wenigstens 8 Kohlenstoffatome aufweisenden geradkettigen oder verzweigten Alkyl- oder Alkenylrest bedeutet;
- R²: eine Kohlenstoff-Kohlenstoffbindung oder einen geradkettigen oder verzweigten Alkylen- oder Alkenylenrest bedeutet;
wobei R¹ und R² zusammen bis zu 30 Kohlenstoffatome, bevorzugt bis zu 20 Kohlenstoffatome, und vorzugsweise 0 bis 3 Doppelbindungen aufweisen;
R³ einen Methylen-, Ethylen- oder n-Propylenrest bedeutet;
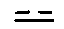 eine Einfach- oder eine Doppelbindung bedeutet;
Z einen Rest der Formel III 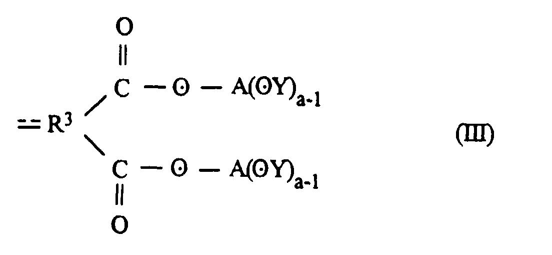 bedeutet,
worin
A, ⊙, a, Y und 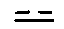 wie oben definiert sind;
c 0 oder 1 bedeutet;
d eine ganze Zahl ≥ 1, bevorzugt 1 - 100, besonders bevorzugt 0 - 20, ganz besonders bevorzugt 0 - 4 bedeutet; und
- b: eine ganze Zahl bedeutet, die wenigstens 1 und höchstens a, das wie oben definiert ist, beträgt;
und deren Molekulargewicht (Gewichtsmittel) vorzugsweise ≤ 100.000 g/mol, bevorzugt ≤ 50.000 g/mol und besonders bevorzugt ≤ 20.000 g/mol beträgt.

Bei den Verbindungen der Formel I bedeuten R¹ und R² bevorzugt zusammen einen geradkettigen Alkyl- bzw. einen Alkenylrest, und für den Fall, daß 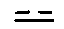 für eine Doppelbindung steht, weisen R¹ und R² keine weitere Doppelbindung auf; sowie für den Fall, daß 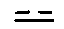 für eine Einfachbindung steht, weisen R¹ und R² insgesamt 1 Doppelbindung auf. Besonders bevorzugt enthalten R¹ und R² zusammen 8 bis 20 Kohlenstoffatome.

Zu den bevorzugten Resten für R¹ zählen n-Octyl, n-Decyl, n-Undecyl, n-Dodecyl, n-Tetradecyl, n-Hexadecyl, n-Octadecyl, Eicosyl, Trimerbutenyl, Diisobutenyl, Tetramerpropenyl, n-Octenyl, n-Decenyl, n-Undecenyl, n-Dodecenyl, n-Tetradecenyl, n-Pentadecenyl, n-Hexadecenyl, n-Heptadecenyl, n-Octadecenyl und Eicosenyl, wobei n-Octenyl, n-Decenyl, n-Dodecenyl und n-Tetradecenyl besonders bevorzugt sind.

Bevorzugt steht R² für einen Rest der Formel

R¹...-CH(Z)₁-CH=CH-

oder

R¹...-CH=CH-CH(Z)₁-CH₂-

wobei R¹ und Z wie oben definiert sind,
und besonders bevorzugt für eine Kohlenstoff-Kohlenstoff-Bindung

Ganz besonders bevorzugt sind Verbindungen der Formel IV 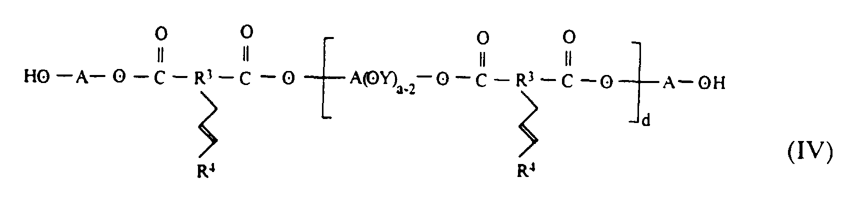 worin
- A, ⊙, R³, Y und d: wie oben definiert sind, und
- R⁴: einen geradkettigen Alkylrest mit 5 bis 27, bevorzugt mit 5 bis 17 Kohlenstoffatomen, besonders bevorzugt einen n-Pentyl-, n-Heptyl-, n-Nonyl oder n-Undecylrest bedeutet.

Vorzugsweise ist R⁴ ausgewählt aus n-Pentyl, n-Heptyl, n-Octyl, n-Nonyl, n-Undecyl, n-Tridecyl, n-Pentadecyl und n-Heptadecyl, besonders bevorzugt aus n-Pentyl, n-Heptyl, n-Nonyl und n-Undecyl. R⁴ kann auch innerhalb eines Moleküls der Formel IV für verschiedene der genannten Reste stehen.

Die erfindungsgemäßen Verbindungen der Formeln I und IV besitzen die gewünschten Eigenschaften als Solubilisatoren, Emulgatoren und/oder waschaktive Substanzen.

Bei den Verbindungen der Formeln I, IV wird der Rest A bevorzugt ausgewählt aus Glycerin, oligomerem Glycerin mit bis zu 20 Glycerineinheiten, wie Diglycerin, Triglycerin, Tetraglycerin, Pentaglycerin, Hexaglycerin, Oktaglycerin, Decaglycerin, sowie aus Pentaerythrit und Trimethylolpropan und Zuckeralkoholen oder Anhydrozuckeralkoholen, wie insbesondere Sorbit, Mannit, Adonit, Arabit, Xylit, Dulcit, Sorbid, Sorbitan und Erythrit. Besonders bevorzugt sind Glycerin und die genannten oligomeren Glycerine. Ganz besonders bevorzugt sind oligomere Glycerine mit 2 bis 10 Glycerineinheiten.

Bei den Verbindungen der Formeln I, IV steht R³ bevorzugt für einen Ethylenrest.

Allgemein werden die Verbindungen der Formel I und IV in wäßrigen und wäßrig/ethanolischen Systemen, die wenigstens eine darin schwer- oder unlösliche Substanz enthalten, als Solubilisatoren, Emulgatoren und/oder waschaktive Substanz verwendet. Bevorzugt ist eine Verwendung beispielsweise in kosmetischen Zusammensetzungen, in Wasch- und Reinigungsmitteln, in pharmazeutischen Zusammensetzungen, in diätetischen und nicht-diätetischen Lebensmitteln und/oder in Pflanzenschutzmitteln.

Somit ist ein weiterer Gegenstand der vorliegenden Erfindung eine kosmetische Zusammensetzung, umfassend wenigstens eine in Wasser schwer- oder unlösliche Substanz und gegebenenfalls übliche Hilfs- und/- oder Zusatzstoffe, wobei die kosmetische Zusammensetzung wenigstens eine der oben genannten Verbindungen der Formel I oder IV insbesondere als Solubilisator und/oder Emulgator umfaßt.

Dabei eignen sich die Verbindungen der Formeln I und IV besonders zur Solubilisierung schwerlöslicher kosmetischer Grundstoffe, insbesondere kosmetischer Öle. Sie besitzen ein ausgezeichnetes Solubilisierungsvermögen für fette Öle wie Erdnußöl, Jojobaöl, Kokosnußöl, Mandelöl, Olivenöl, Palmöl, Ricinusöl, Sojaöl oder Weizenkeimöl; oder für ätherische Öle wie Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamomöl, Pfefferminzöl, Kampferöl usw.; für kosmetische Grundstoffe wie Galaxolid, Vanillin, Menthol, Hexylzimtaldehyd, Benzylacetat, Lysmeral, Linalool, Geraniol, Linalylacetat, usw.; oder für Mischungen aus diesen Ölen bzw. Grundstoffen.

Bei den erfindungsgemäßen kosmetischen Zusammensetzungen handelt es sich um Solubilisate auf Wasser- oder Wasser/Alkoholbasis. Vorzugsweise werden die Verbindungen der Formeln I und IV als Solubilisator und/oder Emulgator im Gewichtsverhältnis von 0,01:1 bis 10:1, vorzugsweise von 0,1 : 1 bis 6 : 1, zum kosmetischen Grundstoff eingesetzt.

Zusätzlich können in der erfindungsgemäßen kosmetischen Zusammensetzung weitere Hilfs- und/oder Zusatzstoffe vorhanden sein. Diese können typische nichtionische, kationische, anionische oder amphotere Tenside sein, wie beispielsweise Alkylpolyglycoside, Fettalkoholsulfate, Fettalkoholethersulfate, Alkansulfonate, Fettalkoholethoxilate, Fettalkoholphosphate, Fettalkoholethersulfonate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester usw. Diese wirken üblicherweise als Coemulgatoren. Als weitere Bestandteile können natürliche oder synthetische Verbindungen, wie beispielsweise Lanolinderivate, Cholesterinderivate, Isopropylmyristat, Isopropylpalmitat, sowie Elektrolyte, Farbstoffe, Konservierungsmittel, Säuren (beispielsweise Milchsäure oder Zitronensäure) und Basen zugesetzt werden.

Als Beispiele für erfindungsgemäße kosmetische Zusammensetzungen seien Badezusatzpräparate wie Badeöle, sowie Rasierwässer, Gesichtswässer, Mundwässer, Haarwässer, Eau de Cologne, Eau de Toilette usw. genannt.

Ein weiterer erfindungsgemäßer Gegenstand ist ein Wasch- bzw. Reinigungsmittel insbesondere zur Oberflächen-, Gewebe- und/oder Körperreinigung, gegebenenfalls umfassend übliche waschaktive Substanzen sowie übliche Hilfs- und/oder Zusatzstoffe, wobei das Wasch- bzw. Reinigungsmittel wenigstens eine der oben genannten Verbindungen der Formel Ia oder IVa insbesondere als waschaktive Substanz umfaßt.

Die erfindungsgemäßen Wasch- und Reinigungsmittel sind beispielsweise anwendbar für Reinigungsprozesse in Technik und Haushalt, für die Textilwäsche, für Reinigungsprozesse im Nahrungsmittelbereich, in Handgeschirrspülmitteln oder für Körperreinigungsmittel, wie z.B. Haarshampoos, Haarspülungen, Dusch- und Schaumbäder. Vorzugsweise enthält das erfindungsgemäße Reinigungsmittel die waschaktive Substanz der Formel I oder IV zu 0,1 bis 90 Gew.-%, bevorzugt zu 0,5 bis 30 Gew.-%. Die erfindungsgemäßen Reinigungsmittel können beliebige weitere anionische, kationische, nichtionische oder amphotere Tenside enthalten, wie z.B. die oben bei den kosmetischen Zusammensetzungen erwähnten Tenside.

Ein Vorteil der Verbindungen der Formel I und IV ist deren niedrige Grenzflächenspannung. Diese trägt zu deren Waschaktivität bei.

Einen weiteren Gegenstand der vorliegenden Erfindung stellen pharmazeutische Zusammensetzungen dar, umfassend wenigstens einen in Wasser schwer- oder unlöslichen Wirk-, Hilfs- oder Zusatzstoff, sowie gegebenenfalls weitere Wirk-, Hilfs- und/oder Zusatzstoffe, wobei die pharmazeutische Zusammensetzung wenigstens eine der oben genannten Verbindungen der Formel I oder IV insbesondere als Solubilisator und/oder Emulgator umfaßt.

Der in Wasser schwer- oder unlösliche Wirkstoff kann beispielsweise ein öllösliches Vitamin oder Vitamin-Derivat sein. Insbesondere sind hier die Vitamine der A-, D-, E- und K-Reihe zu nennen.

In den erfindungsgemäßen pharmazeutischen Zusammensetzungen wirken die Verbindungen der Formel I und IV insbesondere als Solubilisator bzw. Emulgator, so daß wäßrige oder wäßrig/alkoholische Wirkstofflösungen zur oralen bzw. topischen Anwendung hergestellt werden können. Einen großen Vorteil stellen hier zudem der geringe Eigengeschmack sowie der nur schwache Eigengeruch der genannten Verbindungen dar.

Neben den genannten Vitaminen, die erfindungsgemäß von den Verbindungen der Formel I bzw. IV bevorzugt in wäßriger Lösung solubilisiert bzw. emulgiert werden, können in wäßrig/alkoholischen Lösungen besonders gut ätherische Öle solubilisiert bzw. emulgiert werden. Auch andere hydrophobe Arzneiwirkstoffe (beispielsweise Miconazol, Hexetidin, Clotrimazol und Benzocain) lassen sich mit den genannten Verbindungen in wäßrige Lösungen überführen.

Die erfindungsgemäßen pharmazeutischen Zusammensetzungen enthalten Verbindungen der Formel I bzw. IV vorzugsweise zu 0,1 bis 90 Gew.-%, bevorzugt zu 0,5 bis 50 Gew.-%, besonders bevorzugt zu 2 bis 25 Gew.-%.

Damit beispielsweise öllösliche Vitamine klare, wäßrige Lösungen ergeben, müssen sie zunächst mit der als Solubilisator bzw. Emulgator wirksamen Verbindung der Formel I bzw. IV innig vermischt werden.

Weitere Reaktionsbedingungen sind den entsprechenden Beispielen zu entnehmen.

Gegenstand der Erfindung ist weiterhin ein diätetisches oder nicht-diätetisches Lebensmittel, umfassend wenigstens einen in Wasser schwer- oder unlöslichen Nähr-, Hilfs- oder Zusatzstoff, wobei das Lebensmittel wenigstens eine der oben genannten Verbindungen der Formel I oder IV insbesondere als Solubilisator und/oder Emulgator umfaßt. Solche Lebensmittel enthalten die genannten Verbindungen vorzugsweise zu 0,01 bis 30 Gew.-%, bevorzugt zu 0,1 bis 10 Gew.-%. Die solubilisierenden bzw. emulgierenden Eigenschaften der Verbindungen der Formel I bzw. IV insbesondere bei ansonsten schwer- oder unlöslichen Nährstoffen schaffen beispielsweise die Voraussetzungen für eine effektive Verwertung der Nährstoffe. Denkbar sind hier beispielsweise Lebensmittel, insbesondere Energie-Getränke, für Leistungssportler. Diese vorteilhaften Eigenschaften der genannten Verbindungen können auch in Tiernahrung und in Tierarzneimitteln nutzbar gemacht werden, beispielsweise in Zusammenhang mit der Verwendung von Fettstoffen in Mischfutter oder zur Herstellung von Lebertranemulsionen in der Veterinärmedizin.

Schließlich ist ein Gegenstand der Erfindung ein Pflanzenschutzmittel, umfassend wenigstens einen in Wasser schwer- oder unlöslichen Wirk-, Hilfs- oder Zusatzstoff, dadurch gekennzeichnet, daß das Pflanzenschutzmittel wenigstens eine der oben genannten Verbindungen der Formel I oder IV insbesondere als Solubilisator und/oder Emulgator umfaßt. Pflanzenschutzmittel enthalten häufig hydrophobe Wirkstoffe, die durch die solubilisierenden bzw. emulgierenden Eigenschaften der genannten Verbindungen besser in wäßrigen Systemen, wie Spritzbrühen, nutzbar gemacht werden können. Die Verbindungen der Formel I bzw. IV werden in Pflanzenschutzmitteln vorzugsweise zu 0,1 bis 80 Gew.-%, besonders bevorzugt zu 1 bis 50 Gew.-% eingesetzt.

Allgemein gesagt können die Verbindungen der Formel I bzw. IV vorteilhaft insbesondere dort eingesetzt werden, wo eine gute Wirkung als Solubilisator bzw. Emulgator erwünscht ist.

Weiterhin bevorzugt ist die erfindungsgemäße Verwendung der in den Beispielen genannten Bisester der Formel I bzw. IV.

Ein weiterer Gegenstand der Erfindung betrifft ein Verfahren zur Herstellung von Verbindungen der Formel I, wobei man eine Verbindung der Formel V 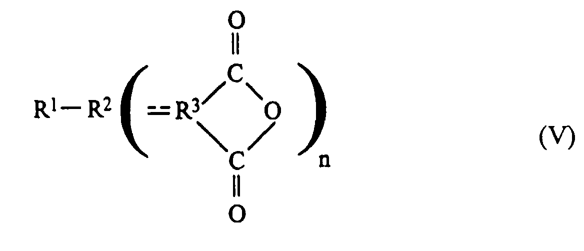 worin
R¹, R² und R³ wie bei den Verbindungen der Formel I definiert sind, und
n für 1 oder 2 steht;
oder
eine Verbindung der Formel VI 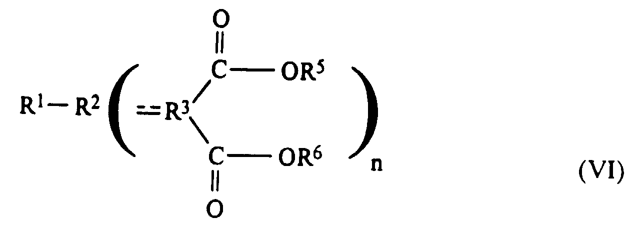 worin
- R¹, R², R³ und n: wie oben definiert sind, und
- R⁵ und R⁶: jeweils unabhängig voneinander für ein Wasserstoffatom oder
einen Alkylrest, insbesondere einen C₁-C₄-Alkylrest stehen;
mit einem monomeren Polyalkohol oder einem oligomeren Polyalkohol der Formel VII

A(⊙H)ₐ (VII)

worin A, ⊙ und a wie oben definiert sind,
in Gegenwart wenigstens eines Katalysators oder eines Gemisches aus mehreren Katalysatoren, wobei der Katalysator bzw. die Katalysatoren bevorzugt ein schwach saurer Katalysator, ein Lewis-Säure-Katalysator und/oder ein basischer Katalysator sind,
umsetzt.

Bei dem Verfahren handelt es sich um eine Veresterungsreaktion. Es wurde überraschend festgestellt, daß die Verwendung von Standard-Veresterungskatalysatoren, wie p-Toluolsulfonsäure, Schwefelsäure, Kaliumhydrogensulfat oder Salzsäure nur bedingt für diese Reaktion geeignet sind, da sie häufig zu hochviskosen Produkten führen, die nicht mehr rührbar sind, sich somit nicht vollständig umsetzen lassen und nicht erfindungsgemäß verwendbar sind.

Erfindungsgemäß bevorzugt ist vielmehr die Verwendung der folgenden Katalysatoren, mit denen sich die oben beschriebenen Nachteile nicht ergeben und die Umsätze von 95 bis 100% bezogen auf die eingesetzten Dicarbonsäurederivate der Formeln V und VI ergeben:
- schwach saure Katalysatoren: beispielsweise phosphorige Säure, hypophosphorige Säure, Phosphorsäure, Phosphonsäure und Borsäure;
- Lewis-Säure-Katalysatoren: beispielsweise Aluminiumchlorid, Bortrifluorid, Orthotitanat, bevorzugt Tetraethylorthotitanat oder Tetrabutylorthotitanat, Zinndioxid und Zinndibutyldilaurat;
- basische Katalysatoren: beispielsweise Natriummethylat, Natriumethylat, Natriumcarbonat, Kaliumcarbonat, Kalium-tert.-butylat, Natriumhydroxid, Kaliumhydroxid, Lithiumhydroxid, Calciumhydroxid, Magnesiumoxid, Kaliumphosphat.

Die genannten Katalysatoren können allein oder bevorzugt als Gemisch eingesetzt werden. Ein besonders bevorzugtes Katalysatorgemisch umfaßt ein Orthotitanat, bevorzugt Tetraethyl- oder Tetrabutylorthotitanat, Kaliumcarbonat und hypophosphorige Säure. Ein solches Katalysatorgemisch führt bei hohen Umsätzen der Dicarbonsäurederivate der Formel V oder VI zu farbhellen Produkten, die vorteilhaft erfindungsgemäß verwendet werden können.

Bei dem erfindungsgemäßen Verfahren werden weiterhin vorzugsweise pro Moläquivalent einer Verbindung der Formel V oder VI wenigstens 4 Moläquivalente an OH-Gruppen des oligomeren Polyalkohols der Formel VII eingesetzt. Der Polyalkohol kann auch im großen Überschuß eingesetzt werden. Restlicher Polyalkohol kann nach der Umsetzung durch Phasentrennung abgetrennt werden.

Üblicherweise findet die Umsetzung in Gegenwart der genannten Katalysatoren unter Abdestillieren des Reaktionswassers statt. Die erste Reaktionsstufe führt zum Halbester, der bei den geforderten Reaktionsbedingungen (geeigneter Katalysator, erhöhte Temperatur, gegebenenfalls Vakuum, destillative Abtrennung des Reaktionswassers oder von entstandenem Alkohol) anschließend zum Bisester der Formel I abreagiert.

Optimal ist ein Einsatz der Katalysatoren in einer Menge von 0,1 bis 5 Gew.-%, bevorzugt 0,5 bis 2 Gew.-%, bezogen auf das Gewicht der Summe der Ausgangsstoffe. Die Reaktion kann in Lösungsmitteln oder lösungsmittelfrei durchgeführt werden. Die Lösungsmittel sollten üblicherweise polar und bei den Reaktionsbedingungen inert sein. Als Lösungsmittel eignen sich beispielsweise N,N-Dimethylformamid, N,N-Dimethylacetamid, N-Methylpyrrolidon, Diglyme, Dimethylethylenglykol, Tetrahydrofuran, Dioxan, Acetonitril, Nitromethan, Hexamethylphosphorsäuretriamid, Dimethylsulfoxid, Ethylencarbonat, Propylencarbonat, Tetrahydrothiophen-1,1-dioxid. Das Lösungsmittel kann während der Umsetzung oder nach Beendigung der Umsetzung abdestilliert werden.

Die Reaktion wird in der Regel bei einem Druck von 1 mbar bis Normaldruck, bevorzugt 20 mbar bis Normaldruck, und bei einer Temperatur von 60 bis 250 °C, bevorzugt von 120 bis 200 °C, durchgeführt.

Das gebildete Reaktionswasser sollte während der Reaktion entfernt werden. Die Reaktionszeiten liegen je nach Bedingungen bei etwa 2 bis etwa 20 Stunden. Die Reaktionskontrolle erfolgt mittels IR-Spektroskopie und/oder über die Bestimmung der Säurezahl.

Die erfindungsgemäßen Verbindungen der Formel I können auch durch das zu ihrer Herstellung verwendete Verfahren definiert werden.

Somit ist ein weiterer Gegenstand der Erfindung eine Verbindung, die aufgrund des oben beschriebenen Verfahrens erhältlich ist.

Die Reaktion muß nicht vollständig ablaufen, um ein erfindungsgemäß einzusetzendes Reaktionsprodukt zu liefern. Bis zu 10 % nicht umgesetzte Monoester der erfindungsgemäßen Verbindungen können toleriert werden. Die nicht vollständig umgesetzten Monoester können auch mit Basen neutralisiert werden und dann als Salz vorliegen, das zusammen mit den vollständig umgesetzten Bisestern verwendet werden kann.

Die erfindungsgemäßen Wirkungen als Solubilisator, Emulgator und/oder waschaktive Substanz sind sowohl mit den Einzelverbindungen der Formel I bzw. IV (Oligomere) als auch mit Gemischen derselben erzielbar. Die Einzelverbindungen können beispielsweise durch chromatographische Trennung der technischen Reaktionsgemische, die als Produkt des erfindungsgemäßen Verfahrens entstehen, erhalten werden, z.B. durch präparative Gelpermeationschromatographie (GPC). Aus Kostengründen werden jedoch bevorzugt die erhaltenen Reaktionsgemische eingesetzt. Durch Variation der Herstellbedingungen, z.B. Überschuß an Alkohol, kann das Molverhältnis der entstandenen Produkte variiert werden.

Die als Ausgangssubstanzen zur Synthese der Verbindungen der Formel I verwendeten Verbindungen der Formeln V und VI lassen sich auf bekannte, übliche Weise herstellen.

Beispielsweise können Alkenylbernsteinsäureanhydride über eine En-Reaktion von Maleinsäureanhydrid mit entsprechenden Olefinen hergestellt werden. Der Maleinierungsgrad kann dabei bis zu 200% betragen, d.h. daß durch 2-fache En-Reaktion 2 Bernsteinsäurereste an das ursprüngliche Olefin gebunden sind, was zu Verbindungen der Formel I mit c = 1 führt. Alkylbernsteinsäuren und -anhydride können beispielsweise durch Hydrierung der entsprechenden Alkenylbernsteinsäureanhydride hergestellt werden. Bei der En-Reaktion können verschiedene isomere Produkte gebildet werden, was beispielhaft anhand des folgenden Reaktionsschemas zur Herstellung von Bernsteinsäure-Derivaten dargestellt ist:

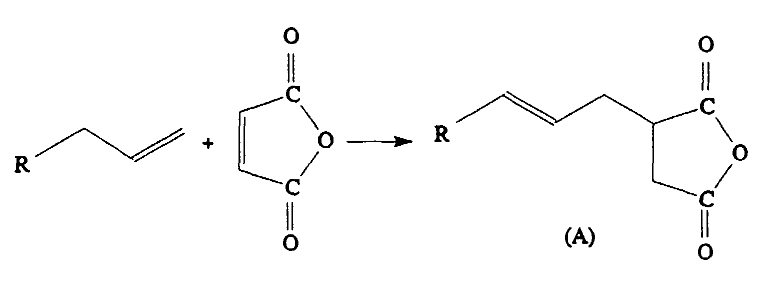

Das Mono-Alkenylbernsteinsäureanhydrid (A) kann aufgrund einer doppelten En-Reaktion weiterreagieren zu den isomeren Produkten (B) und (C), die Bis-Alkenylbernsteinsäureanhydride darstellen.

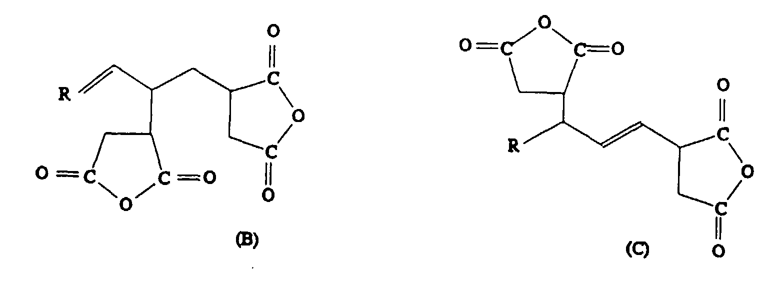

Die Verbindungen der Formeln (A), (B) und (C) stehen dabei beispielhaft für die möglichen cis-/trans-Isomere, die bezüglich der Doppelbindung entstehen können.

Bei einer solchen Reaktion entstehen die Verbindungen der Formel (A) im allgemeinen als Hauptprodukt, während die Verbindungen der Formeln (B) und (C) nur zu jeweils ca. 5% entstehen. Durch die Wahl geeigneter Reaktionsbedingungen kann das Verhältnis der einzelnen Produkte zueinander verschoben werden. Die genannten Produkte können als technisches Gemisch oder, nach ihrer Isolierung, in reiner Form als Ausgangssubstanzen im erfindungsgemäßen Verfahren eingesetzt werden.

Alkenylbernsteinsäureester können durch Veresterung der Alkenylbernsteinsäureanhydride mit Alkoholen wie z.B. Methanol, Ethanol, Propanol, i-Propanol, Butanol oder i-Butanol erhalten werden. Diese Veresterung kann sauer katalysiert werden. Günstig ist ein Überschuß an Alkohol, der anschließend wieder abdestilliert wird.

Die Alkylmalonsäureester bzw. -anhydride und die Alkylglutarsäureester bzw. -anhydride der Formel VI können durch Umsetzung der entsprechenden Malon- bzw. Glutarsäurederivate mit Alkylhalogeniden, Alkylsulfaten oder Alkyltoxilaten unter basischer Katalyse, wie beispielsweise einem Alkoholat, hergestellt werden.

Die Alkenylmalonsäure- und -glutarsäureester bzw. -anhydride können analog den Alkylmalonsäurederivaten bzw. Alkylglutarsäurederivaten durch Einsatz entsprechender Alkenylhalogenide, Alkenylsulfate oder Alkenyltosilate hergestellt werden.

Die entsprechenden Dicarbonsäurederivate der Formel VI entstehen aus den Estern durch Hydrolyse. Durch partielle Hydrolyse sind die entsprechenden Monocarbonsäuremonoesterderivate erhältlich.

Durch Umsetzung der genannten Ester mit Aldehyden unter basischer Katalyse werden die entsprechenden Alkylidenderivate erhalten. Diese können ebenfalls zu den Alkylderivaten hydriert werden.

Im folgenden sind Synthesebeispiele für Verbindungen der Formel I sowie Anwendungsbeispiele für erfindungsgemäße Zusammensetzungen aufgeführt.

### Beispiele

Die Gegenstände der vorliegenden Erfindung werden durch die nachfolgenden Beispiele erläutert, in denen weitere bevorzugte Einzelmerkmale der Erfindung beschrieben sind.

### 1. Synthesebeispiele

Nachfolgend sind Synthesebeispiele für einige der erfindungsgemäßen Verbindungen der Formel I bzw. IV sowie für Vergleichsverbindungen des Standes der Technik aufgeführt.

### Allgemeine Herstellungsverfahren:

### Variante A:

Ein Alkenylbernsteinsäureanhydrid wird zusammen mit einem Polyalkohol vorgelegt. Gleichzeitig werden 0,5 Gew.-% hypophosphorige Säure als Katalysator zugegeben. Unter mäßigem Stickstoffstrom wird die Mischung auf 180 °C aufgeheizt. Das Reaktionswasser wird unter Rühren abdestilliert. Der Reaktionsverlauf wird über die Säurezahl bestimmt. Wenn die Säurezahl < 10 mg KOH/g beträgt, werden die Reaktionsmischung abgekühlt und die restlichen Säuregruppen mit NaOH neutralisiert.

### Variante B:

Ein Alkenylbernsteinsäureanhydrid wird zusammen mit einem Polyalkohol vorgelegt. Gleichzeitig werden 1 Gew.-% Natriummethylat als Katalysator zugegeben. Unter mäßigem Stickstoffstrom wird die Mischung auf 120 °C aufgeheizt, und es wird ein Vakuum von 20 mbar angelegt. Das Reaktionswasser wird unter Rühren abdestilliert. Der Reaktionsverlauf wird über die Säurezahl bestimmt. Wenn die Säurezahl < 10 mg KOH/g beträgt, werden die Reaktionsmischung abgekühlt und die restlichen Säuregruppen mit NaOH neutralisiert.

### Variante C:

Ein Polyalkohol wird zusammen mit dem Katalysatorgemisch (0,5 Gew.-% hypophosphorige Säure, 0,5 Gew.-% Tetraethylorthotitanat und 0,2 Gew.-% Natriumcarbonat) vorgelegt. Bei einer Temperatur von 80 °C wird innerhalb einer Stunde das Alkenylbernsteinsäureanhydrid zugetropft. Anschließend wird die Reaktionsmischung unter einem mäßigen Stickstoffstrom auf 180 °C aufgeheizt. Das Reaktionswasser wird unter Rühren abdestilliert. Der Reaktionsverlauf wird über die Säurezahl kontrolliert. Wenn die Säurezahl < 10 mg KOH/g beträgt, werden die restlichen Säuregruppen mit NaOH neutralisiert. Die noch warme Reaktionsmischung wird filtriert und anschließend abgekühlt.

### Variante D (Herstellung von Vergleichsverbindungen):

Ein Alkenylbernsteinsäureanhydrid wird zusammen mit einem Polyalkohol vorgelegt. Unter mäßigem Stickstoffstrom wird die Mischung auf 80 °C aufgeheizt und gerührt. Der Reaktionsverlauf wird IR-spektroskopisch verfolgt. Nach Abnahme der Anhydridbanden bei 1780 und 1830 cm⁻¹ wird die Reaktionsmischung abgekühlt. Die freien Säuregruppen können mit NaOH neutralisiert werden.

### Beispiel 1

Herstellung eines Gemisches von monomeren und oligomeren Bisestern, ausgehend von einem C8- und einem C10-Alken-(2)-yl-(1)-bernsteinsäureanhydrid (Molverhältnis C8 : C10 = 1 : 1) und Polyglycerin (mittleres Molekulargewicht des Polyglycerins = 201 g/mol; Molverhältnis Alkenylbernsteinsäureanhydrid zu Polyglycerin 1:2). Die Herstellung erfolgte mit Hilfe des Verfahrens der Variante A; die Säurezahl des Endproduktes betrug 7,2 mg KOH/g.

### Beispiel 2

Herstellung eines Gemisches von monomeren und oligomeren Bisestern, ausgehend von einem C10- und einem C12-Alken-(2)-yl-(1)-bernsteinsäureanhydrid (Molverhältnis C10 : C12 = 0,5 : 1) und Polyglycerin (mittleres Molekulargewicht des Polyglycerins = 201 g/mol; Molverhältnis Alkenylbernsteinsäureanhydrid zu Polyglycerin 1:2). Die Herstellung erfolgte mittels des Verfahrens der Variante A; die Säurezahl betrug 7,5 mg KOH/g.

### Beispiel 3

Herstellung eines Gemisches von monomeren und oligomeren Bisestern, ausgehend von einem C12- und einem C14-Alken-(2)-yl-(1)-bernsteinsäureanhydrid (Molverhältnis C12 : C14 = 1 : 0,5) und Polyglycerin (mittleres Molekulargewicht des Polyglycerins = 201 g/mol; Molverhältnis Alkenylbernsteinsäureanhydrid zu Polyglycerin 1:2). Die Herstellung erfolgte mittels des Verfahrens der Variante A; die Säurezahl betrug 7,6 mg KOH/g.

### Beispiel 4

Herstellung eines Gemisches von monomeren und oligomeren Bisestern, ausgehend von einem C12- und einem C14-Alken-(2)-yl-(1)-bernsteinsäureanhydrid (Molverhältnis C12 : C14 = 1 : 0,5) und Pentaglycerin (mittleres Molekulargewicht des Polyglycerins = 388 g/mol, Molverhältnis Alkenylbernsteinsäureanhydrid zu Polyglycerin 1:2). Die Herstellung erfolgte mittels des Verfahrens der Variante A; die Säurezahl betrug 3.9 mg KOH/g.

### Beispiel 5

Herstellung eines Gemisches von monomeren und oligomeren Bisestern, ausgehend von einem Decen-(2)-yl-(1)-bernsteinsäureanhydrid und Mannit (Molverhältnis Alkenylbernsteinsäureanhydrid zu Mannit 1:2). Die Herstellung erfolgte mittels des Verfahrens der Variante B; die Säurezahl betrug 13,5 mg KOH/g.

### Beispiel 6

Herstellung eines Gemisches von monomeren und oligomeren Bisestern, ausgehend von einem Dodecen-(2)-yl-(1)-bernsteinsäureanhydrid und Sorbit (Molverhältnis Alkenylbernsteinsäureanhydrid zu Sorbit 1:2). Die Herstellung erfolgte mittels des Verfahrens der Variante B; die Säurezahl betrug 15 mg KOH/g.

### Beispiel 7

Herstellung eines Gemisches von monomeren und oligomeren Tetraestern, ausgehend von einem C12- und einem C14-Alkenyl-bis-bernsteinsäureanhydrid (Molverhältnis C12 : C14 = 1 : 0,5) und Glycerin (Molverhältnis Alkenyl-bisbernsteinsäureanhydrid zu Glycerin 1:4). Die Herstellung erfolgte mittels des Verfahrens der Variante A; die Säurezahl betrug 17 mg KOH/g.

### Beispiel 8

Herstellung eines Gemisches von monomeren und oligomeren Bisestern, ausgehend von einem Decen-(2)-yl-(1)-bernsteinsäureanhydrid und Polyglycerin (mittleres Molekulargewicht des Polyglycerins = 201 g/mol, Molverhältnis Alkenylbernsteinsäureanhydrid zu Polyglycerin 1 : 2). Die Herstellung erfolgte mittels des Verfahrens der Variante C; die Säurezahl betrug 7,1 mg KOH/g.

### Beispiel 9

Herstellung eines Gemisches von monomeren und oligomeren Bisestern, ausgehend von einem Octadecen-(2)-yl-(1)-bernsteinsäureanhydrid und Decaglycerin (mittleres Molekulargewicht des Polyglycerins = 750 g/mol, Molverhältnis Alkenylbernsteinsäureanhydrid zu Polyglycerin 1 : 2). Die Herstellung erfolgte mittels des Verfahrens der Variante A; die Säurezahl betrug 9,1 mg KOH/g.

### Beispiel 10 (Vergleichsverbindung)

Herstellung eines Monoesters, ausgehend von einem C10-C12-Alken-(2)-yl-(1)-bernsteinsäureanhydrid (Molverhältnis C10 : C12 = 0,5 : 1) und Polyglycerin (mittleres Molekulargewicht des Polyglycerins = 201 g/mol; Molverhältnis Alkenylbernsteinsäureanhydrid zu Polyglycerin 1 : 2). Die Herstellung erfolgte mittels des Verfahrens der Variante D; die Säurezahl betrug 126 mg KOH/g.

### Beispiel 11 (Vergleichsverbindung)

Herstellung des Natriumsalzes der Verbindung aus Beispiel 10 (mittleres Molekulargewicht des Polyglycerins = 201 g/mol; Molverhältnis Alkenylbernsteinsäureanhydrid zu Polyglycerin 1:2). Die Herstellung erfolgte mit Hilfe des Verfahrens der Variante D; nach der Neutralisation betrug die Säurezahl < 1 mg KOH/g.

### 2. Anwendungsbeispiele

### 2.1 Wirksamkeit der erfindungsgemäßen Verbindungen:

In Tabelle 1 sind die Hartwasserbeständigkeit und die Grenzflächenspannungen gegen ausgewählte Öle aus dem Kosmetik- und Reinigungssektor dargestellt.

Für die Verwendung der Produkte als waschaktive Substanzen in Reinigungsmitteln ist die Wasserhärtestabilität ein wichtiges Anforderungskriterium. Getestet wurde die Löslichkeit in Wasser mit 20° dH bei 25 °C; die Konzentration betrug 1 g/l.

Das Emulgier- bzw. Solubilisierungsvermögen der erfindungsgemäßen Substanzen wird durch die Grenzflächenspannung zwischen der wäßrigen Lösung (dest. Wasser) der Substanz und einer Ölphase charakterisiert. Die Messung erfolgte durch ein Spinning Drop Tensiometer.

**Tabelle 1**

| **Produkt aus Synthesebeispiel Nr.** | **Löslichkeit in H**_{**2**}**O (25°C)** | **Löslichkeit in H**_{**2**}**O 20°dH (25°C)** | **Grenzflächenspannungen**^{**a)**} **gegen** | |
|---|---|---|---|---|
| | | | **Olivenöl** | **Rosmarinöl** |
| 1 | klar | klar | 0,93 mN/m | 0,18 mN/m |
| 2 | klar | klar | 0,66 mN/m | 0,15 mN/m |
| 3 | klar | klar | 0,88 mN/m | 0,12 mN/m |
| 4 | klar | klar | | |
| 5 | klar | Spur trüb | 0,93 mN/m | 1,3 mN/m |
| 6 | klar | Spur trüb | 1,34 mN/m | 2,28 mN/m |
| 7 | klar | Spur trüb | | |
| 8 | klar | klar | 0,75 mN/m | 0,16 mN/m |
| 9 | klar | klar | | |
| 10 | leicht trüb | trüb | 2,8 mN/m | 2,0 mN/m |
| 11 | klar | trüb | 2,3 mN/m | 2,9 mN/m |

| | | | | |
|---|---|---|---|---|
| ^{a)}Konzentration = 1 g/l, bei 25°C gemessen | | | | |

Es zeigt sich, daß die erfindungsgemäßen Verbindungen der Synthesebeispiele 1 bis 9 eine bessere Hartwasserbeständigkeit und/oder wesentlich geringere Grenzflächenspannungen als die Verbindungen der Synthesebeispiele 10 und 11, die zum Vergleich dienen und aus der JP 05 125 014 bekannt sind, aufweisen. Dies erlaubt die bevorzugte Verwendung der erfindungsgemäßen Verbindungen als waschaktive Substanz und/oder als Emulgator/Solubilisator.

### 2.2 Herstellung von Solubilisaten von kosmetischen Ölen als Beispiele für erfindungsgemäße kosmetische Zusammensetzungen

Die Verbindungen der Formel I und IV können als reine Substanz oder als wäßrige Lösung eingesetzt werden. Üblicherweise werden 1 bis 6 g (bezogen auf die Wirksubstanz) des entsprechenden Bisesters mit 1 g des jeweils verwendeten ätherischen Öls bzw. Parfumöls innig gemischt, beispielsweise mittels eines Magnetrührers. Unter ständigem Rühren wird mit einer Bürette langsam demineralisiertes Wasser ad 100 g zugefügt. Bei Bedarf werden die Mischungen auf 60 bis 80 °C erwärmt.

Eine verbreitete Methode zur Auffindung der optimalen Solubilisationswirkung von amphiphilen Verbindungen stellt die Trübungstitration dar, wie sie z.B. bei A. Domsch, Die kosmetischen Präparate, 4. Auflage des von G.A. Novak begründeten Werkes, Band II, Wäßrige und tensidhaltige Formulierungen, beschrieben worden ist.

Die Anwendungsbeispiele 1 bis 10 zeigen die vorteilhaften Eigenschaften der erfindungsgemäßen kosmetischen Zusammensetzungen, während die Vergleichsbeispiele 1 bis 4 die Nachteile hinsichtlich der Stabilität bei Vergleichsverbindungen belegen.

### Anwendungsbeispiel 1

1 g Mundpflegearomaöl Dragoco ZM 0065 und 3 g der Verbindung des Synthesebeispiels 1 werden mit 96 g Wasser vermischt und bei Raumtemperatur 5 Minuten gerührt. Man erhält ein klares Solubilisat, das über einen langen Zeitraum stabil ist.

### Anwendungsbeispiel 2

1 g Lavendelöl und 3 g der Verbindung des Synthesebeispiels 2 werden mit 96 g Wasser vermischt und bei Raumtemperatur 5 Minuten gerührt. Man erhält eine klare Lösung, die über einen langen Zeitraum stabil ist.

### Anwendungsbeispiel 3

1 g Rosmarinöl und 3 g der Verbindung des Synthesebeispiels 2 werden mit 96 g Wasser vermischt und bei Raumtemperatur 5 Minuten gerührt. Man erhält eine klare Lösung, die über einen langen Zeitraum stabil ist.

### Anwendungsbeispiel 4

1 g Fichtennadelöl und 3 g der Verbindung des Synthesebeispiels 2 werden mit 76,8 g Wasser und 19,2 g Ethanol vermischt und bei Raumtemperatur 5 Minuten gerührt. Man erhält eine klare Lösung, die über einen langen Zeitraum stabil ist.

### Anwendungsbeispiel 5

1 g Lavendelöl und 3 g der Verbindung des Synthesebeispiels 3 werden mit 76,8 Wasser und 19,2 g Ethanol vermischt und bei Raumtemperatur 5 Minuten gerührt. Man erhält eine klare Lösung, die über einen langen Zeitraum stabil ist.

### Anwendungsbeispiel 6

1 g Latschenkieferöl und 3 g der Verbindung des Synthesebeispiels 3 werden mit 96 Wasser vermischt und bei Raumtemperatur 5 Minuten gerührt. Man erhält eine klare Lösung, die über einen langen Zeitraum stabil ist.

### Anwendungsbeispiel 7

1 g Rosmarinöl und 3 g der Verbindung des Synthesebeispiels 4 werden mit 76,8 g Wasser und 19,2 g Ethanol vermischt und bei Raumtemperatur 5 Minuten gerührt. Man erhält eine leicht trübe Lösung, die über einen langen Zeitraum stabil ist.

### Anwendungsbeispiel 8

1 g Parfümöl Blue Water (Fa. Haarmann & Reimer) und 3 g der Verbindung des Synthesebeispiels 5 werden mit 76,8 g Wasser und 19,2 g Ethanol vermischt und bei Raumtemperatur 5 Minuten gerührt. Man erhält eine klare Lösung, die über einen langen Zeitraum stabil ist.

### Anwendungsbeispiel 9

1 g Mundpflegeöl (Dragoco ZM 0065) und 3 g der Verbindung des Synthesebeispiels 6 werden mit 96 g Wasser vermischt und bei Raumtemperatur 5 Minuten gerührt. Man erhält eine klare Lösung, die über einen langen Zeitraum stabil ist.

### Anwendungsbeispiel 10

1 g Rosmarinöl und 3 g der Verbindung des Synthesebeispiels 7 werden mit 96 g Wasser vermischt und bei Raumtemperatur 5 Minuten gerührt. Man erhält eine klare Lösung, die über einen langen Zeitraum stabil ist.

### Anwendungsbeispiel 11

1 g Fichtennadelöl und 3 g der Verbindung des Synthesebeispiels 8 werden mit 96 g Wasser vermischt und bei Raumtemperatur 5 Minuten gerührt. Man erhält eine klare Lösung, die über einen langen Zeitraum stabil ist.

### Vergleichsbeispiel 1

1 g Rosmarinöl und 3 g der Verbindung des Synthesebeispiels 10 werden mit 96 g Wasser vermischt und bei Raumtemperatur 5 Minuten gerührt. Man erhält eine milchige Emulsion, die sich nach 24 h bereits aufgetrennt hat.

### Vergleichsbeispiel 2

1 g Rosmarinöl und 3 g der Verbindung des Synthesebeispiels 11 werden mit 96 g Wasser vermischt und bei Raumtemperatur 5 Minuten gerührt. Man erhält eine milchige Emulsion, die sich nach 24 h bereits aufgetrennt hat.

### Vergleichsbeispiel 3

1 g Mundpflegearomaöl (Dragoco ZM 0065) und 3 g der Verbindung des Synthesebeispiels 10 werden mit 76,8 g Wasser und 19,2 g Ethanol vermischt und bei Raumtemperatur 5 Minuten gerührt. Man erhält eine milchige Emulsion, die sich nach 24 h bereits aufgetrennt hat.

### Vergleichsbeispiel 4

1 g Mundpflegeöl (Dragoco ZM 0065) und 3 g der Verbindung des Synthesebeispiels 11 werden mit 76,8 g Wasser und 19,2 g Ethanol vermischt und bei Raumtemperatur 5 Minuten gerührt. Man erhält eine milchige Emulsion, die sich nach 24 h bereits aufgetrennt hat.

### 2.3 Herstellung von Solubilisaten von öllöslichen Vitaminen als Beispiel einer erfindungsgemäßen pharmazeutischen Zusammensetzung

### Anwendungsbeispiel 1

5 g Vitamin-A-Palmitat werden mit 25 g der Verbindung des Synthesebeispiels 9 gemischt und auf 60 bis 65 °C erwärmt. Unter innigem Rühren wird in dieses Gemisch sehr langsam 70 g des ebenfalls auf 60 bis 65 °C erwärmten Wassers eingearbeitet. Man erhält dabei ein klares Solubilisat. Erfolgt die Zugabe des Wassers zu schnell, kann die Lösung trüb werden.

## Patentansprüche

1. Verbindung der Formel I
(H⊙)_{a-b}A(⊙X)_{b} (I)
worin
A den Rest eines monomeren Polyalkohols oder eines oligomeren Polyalkohols, der aus bis zu 20 über Etherbindungen miteinander verknüpften Monomeren besteht, bedeutet, wobei der monomere Polyalkohol bzw. das Monomer des oligomeren Polyalkohols ein Alkohol ist, der wenigstens 3 Kohlenstoffatome sowie a OH-Gruppen aufweist;
a ≥ 2 ist;
⊙ jeweils ein Sauerstoffatom einer der OH-Gruppen des Restes A bedeutet;
X einen Rest der Formel II 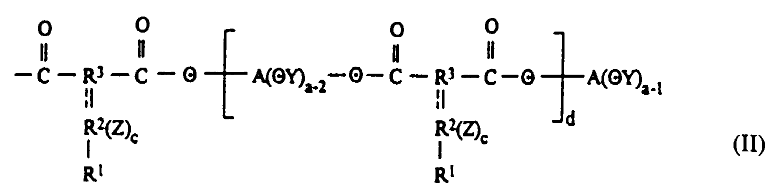
bedeutet;
worin
A, ⊙ und a wie oben definiert sind;
Y ein Wasserstoffatom, oder einen Rest X, der wie oben definiert ist, bedeutet;
R¹ einen wenigstens 8 Kohlenstoffatome aufweisenden geradkettigen oder verzweigten Alkyl- oder Alkenylrest bedeutet;
R² eine Kohlenstoff-Kohlenstoffbindung oder einen geradkettigen oder verzweigten Alkylen- oder Alkenylenrest bedeutet;
wobei R¹ und R² zusammen bis zu 30 Kohlenstoffatome, bevorzugt bis zu 20 Kohlenstoffatome, und vorzugsweise 0 bis 3 Doppelbindungen aufweisen;
R³ einen Methylen-, Ethylen- oder n-Propylenrest, bevorzugt einen Ethylenrest, bedeutet;
-- eine Einfach- oder eine Doppelbindung bedeutet;
z einen Rest der Formel III 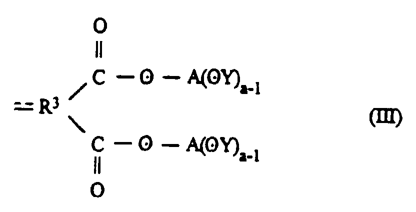
bedeutet,
worin
A, ⊙, a, Y und ―― wie oben definiert sind;
c 0 oder 1 bedeutet;
d eine ganze Zahl ≥ 1, bevorzugt 1 - 100, besonders bevorzugt 1 - 20, ganz besonders bevorzugt 1 - 4 bedeutet;
und
b eine ganze Zahl bedeutet, die wenigstens 1 und höchstens a, das wie oben definiert ist, beträgt;
und deren Molekulargewicht (Gewichtsmittel) vorzugsweise ≤ 100.000 g/mol, bevorzugt ≤ 50.000 g/mol und besonders bevorzugt ≤ 20.000 g/mol beträgt.

2. Verbindung der Formel I gemäß Anspruch 1, worin R¹ und R² zusammen einen geradkettigen Alkyl- bzw. einen Alkenylrest mit insgesamt 8 bis 20 Kohlenstoffatomen bedeuten, und für den Fall, daß ―― für eine Doppelbindung steht, R¹ und R² keine weitere Doppelbindung aufweisen; sowie für den Fall, daß ―― für eine Einfachbindung steht, R¹ und R² insgesamt 1 Doppelbindung aufweisen.

3. Verbindung gemäß einem der Ansprüche 1 oder 2, gekennzeichnet durch die Formel IV 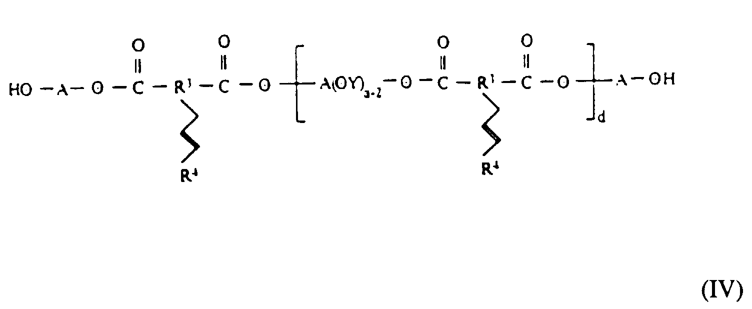 worin
A, ⊙, R³, Y und d wie in Anspruch 1 definiert sind, und
R⁴ einen geradkettigen Alkylrest mit 5 bis 27, bevorzugt mit 5 bis 17, Kohlenstoffatomen, besonders bevorzugt einen n-Pentyl-, n-Heptyl-, n-Nonyl- oder n-Undecylrest bedeutet.

4. Verbindung gemäß einem der Ansprüche 1 bis 3,
worin
der Rest A ausgewählt ist aus Glycerin, oligomerem Glycerin mit bis zu 20 Glycerineinheiten, Pentaerythrit, Trimethylolpropan und Zukkeralkoholen und Anhydrozuckeralkoholen, wie insbesondere Sorbit, Mannit, Adonit, Arabit, Xylit, Dulcit, Sorbid, Sorbitan und Erythrit;
bevorzugt aus Glycerin und oligomerem Glycerin mit bis zu 20 Glycerineinheiten;
besonders bevorzugt aus oligomerem Glycerin mit 2 bis 10 Glycerineinheiten.

5. Verwendung einer Verbindung, wie sie in einem der Ansprüche 1 bis 4 definiert ist, als Solubilisator, Emulgator und/oder waschaktive Substanz.

6. Verwendung gemäß Anspruch 5 in kosmetischen Zusammensetzungen, Wasch- und Reinigungsmitteln, in pharmazeutischen Zusammensetzungen, diätetischen und nicht-diätetischen Lebensmitteln oder Pflanzenschutzmitteln.

7. Kosmetische Zusammensetzung, umfassend wenigstens eine in Wasser schwer- oder unlösliche Substanz und gegebenenfalls übliche Hilfs- und/oder Zusatzstoffe, dadurch gekennzeichnet, daß die kosmetische Zusammensetzung wenigstens eine Verbindung der Formel Ia gemäß einem der Ansprüche 1 bis 4, insbesondere als Solubilisator und/oder Emulgator umfaßt.

8. Wasch- und/oder Reinigungsmittel, insbesondere zur Oberflächen-, Gewebe- und/oder Körperreinigung, gegebenenfalls umfassend übliche waschaktive Substanzen sowie übliche Hilfs- und/oder Zusatzstoffe, dadurch gekennzeichnet, daß das Wasch- bzw. Reinigungsmittel wenigstens eine Verbindung der Formel Ia gemäß einem der Ansprüche 1 bis 4 insbesondere als waschaktive Substanz umfaßt.

9. Pharmazeutische Zusammensetzung, umfassend wenigstens einen in Wasser schwer- oder unlöslichen Wirk-, Hilfs- oder Zusatzstoff, sowie gegebenenfalls weitere Wirk-, Hilfs- und/oder Zusatzstoffe, dadurch gekennzeichnet, daß die pharmazeutische Zusammensetzung wenigstens eine Verbindung der Formel Ia gemäß einem der Ansprüche 1 bis 4 insbesondere als Solubilisator und/oder Emulgator umfaßt.

10. Diätetisches oder nicht-diätetisches Lebensmittel, umfassend wenigstens einen in Wasser schwer- oder unlöslichen Nähr-, Hilfs- oder Zusatzstoff, dadurch gekennzeichnet, daß das Lebensmittel wenigstens eine Verbindung der Formel Ia gemäß einem der Ansprüche 1 bis 4 insbesondere als Solubilisator und/oder Emulgator umfaßt.

11. Pflanzenschutzmittel, umfassend wenigstens einen in Wasser schwer- oder unlöslichen Wirk-, Hilfs- oder Zusatzstoff, dadurch gekennzeichnet, daß das Pflanzenschutzmittel wenigstens eine Verbindung der Formel Ia gemäß einem der Ansprüche 1 bis 4 insbesondere als Solubilisator und/oder Emulgator umfaßt.

12. Verfahren zur Herstellung einer Verbindung der Formel I gemäß einem der Ansprüche 1 oder 4, dadurch gekennzeichnet, daß man eine Verbindung der Formel V 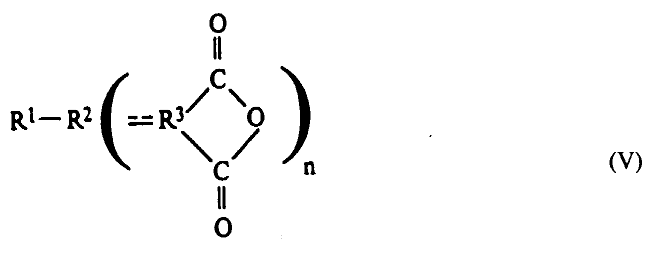 worin
R¹, R² und R³ wie in einem der Ansprüche 1 bis 4 definiert sind, und
n für 1 oder 2 steht;
oder
eine Verbindung der Formel VI 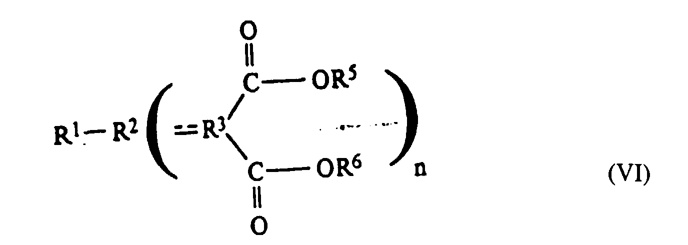 worin
R¹, R², R³ und n wie oben definiert sind, und
R⁵ und R⁶ jeweils unabhängig voneinander für ein Wasserstoffatom oder
einen Alkylrest, insbesondere einen C₁-C₄-Alkylrest stehen;
mit einem monomeren Polyalkohol oder einem oligomeren Polyalkohol der Formel VII
A(⊙H)ₐ (VII)
worin A, ⊙ und a wie in einem der Ansprüche 1 bis 4 definiert sind,
in Gegenwart wenigstens eines Katalysators oder eines Gemisches aus mehreren Katalysatoren, wobei der Katalysator bzw. die Katalysatoren bevorzugt ein schwach saurer Katalysator, ein Lewis-Säure-Katalysator und/oder ein basischer Katalysator sind,
umsetzt.

## Claims

1. A compound of the formula I
(H⊙)_{a-b}A(⊙X)_{b} (I)
where
A is the radical of a monomeric polyalcohol or of an oligomeric polyalcohol which consists of up to 20 monomers linked to one another via ether bonds, the monomeric polyalcohol or the monomer of the oligomeric polyalcohol being an alcohol which has at least 3 carbon atoms and a OH groups;
a ≥ 2;
⊙ is in each case an oxygen atom of one of the OH groups of the radical A;
X is a radical of the formula II 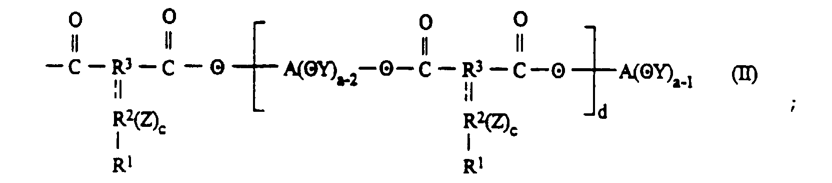
where
A, ⊙ and a are as defined above;
Y is a hydrogen atom, or a radical X, which is defined as above;
R¹ is a straight-chain or branched alkyl or alkenyl radical having at least 8 carbon atoms;
R² is a carbon-carbon bond or a straight-chain or branched alkylene or alkenylene radical;
where R¹ and R² together have up to 30 carbon atoms, preferably up to 20 carbon atoms, and preferably 0 to 3 double bonds;
R³ is a methylene, ethylene or n-propylene radical, preferably an ethylene radical;
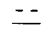 is a single or a double bond;
Z is a radical of the formula III 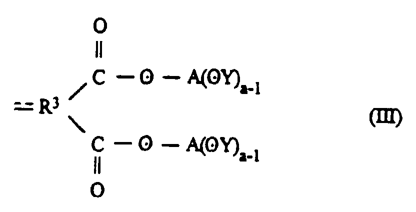
where
A, ⊙, a, Y and 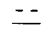 are as defined above;
c is 0 or 1;
d is an integer ≥ 1, preferably 1 - 100, particularly preferably 1 - 20, very particularly preferably 1 - 4;
and
b is an integer which is at least 1 and at most a, which is as defined above;
and whose molecular weight (weight average) is preferably ≤ 100,000 g/mol, preferably ≤ 50,000 g/mol and particularly preferably ≤ 20,000 g/mol.

2. A compound of the formula I as claimed in claim 1, where
R¹ and R² together are a straight-chain alkyl or an alkenyl radical with a total of 8 to 20 carbon atoms, and in the case in which 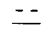 is a double bond, R¹ and R² have no further double bond; and in the case in which 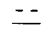 is a single bond, R¹ and R² in total have 1 double bond.

3. A compound as claimed in one of claims 1 and 2, which has the formula IV 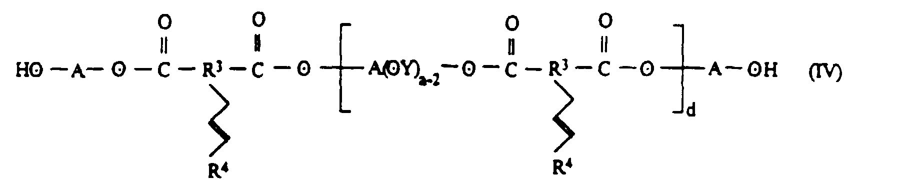 where
A, ⊙, R³, Y and d are as defined above, and
R⁴ is a straight-chain alkyl radical having 5 to 27 carbon atoms, preferably having 5 to 17 carbon atoms, particularly preferably an n-pentyl, n-heptyl, n-nonyl or n-undecyl radical.

4. A compound as claimed in one of claims 1 to 3, where
the radical A is selected from glycerol, oligomeric glycerol having up to 20 glycerol units, pentaerythritol, trimethylolpropane and sugar alcohols and anhydrosugar alcohols, such as, in particular, sorbitol, mannitol, adonitol, arabitol, xylitol, dulcitol, isosorbide, sorbitan and erythritol;
preferably from glycerol and oligomeric glycerol having up to 20 glycerol units;
particularly preferably from oligomeric glycerol having 2 to 10 glycerol units.

5. The use of a compound as defined in one of claims 1 to 4 as a solubilizer, emulsifier and/or detergent.

6. The use as claimed in claim 5 in cosmetic compositions, detergents and cleaners, in pharmaceutical compositions, dietetic and nondietetic foodstuffs or crop protection compositions.

7. A cosmetic composition, comprising at least one substance which is poorly soluble or insoluble in water and, if appropriate, customary auxiliaries and/or additives, wherein the cosmetic composition comprises at least one compound of the formula I as set forth in one of claims 1 to 4, in particular as a solubilizer and/or emulsifier.

8. A detergent and/or cleaner, in particular for surface-, fabric- and/or body-cleaning, if appropriate comprising customary detergents and customary auxiliaries and/or additives, wherein the detergent or cleaner comprises at least one compound of the formula I as set forth in one of claims 1 to 4, in particular, as a detergent.

9. A pharmaceutical composition comprising at least one active compound, auxiliary or additive which is poorly soluble or insoluble in water, and, if appropriate, other active compounds, auxiliaries and/or additives, wherein the pharmaceutical composition comprises at least one compound of the formula I as set forth in one of claims 1 to 4, in particular, as a solubilizer and/or emulsifier.

10. A dietetic or nondietetic foodstuff, comprising at least one foodstuff, auxiliary or additive which is poorly soluble or insoluble in water, wherein the foodstuff comprises at least one compound of the formula I as set forth in one of claims 1 to 4, in particular, as a solubilizer and/or emulsifier.

11. A crop protection composition, comprising at least one active compound, auxiliary or additive which is poorly soluble or insoluble in water, wherein the crop protection composition comprises at least one compound of the formula I as set forth in one of claims 1 to 4, in particular, as a solubilizer and/or emulsifier.

12. A process for preparing a compound of the formula I as claimed in one of claims 1 and 4, which comprises reacting a compound of the formula V 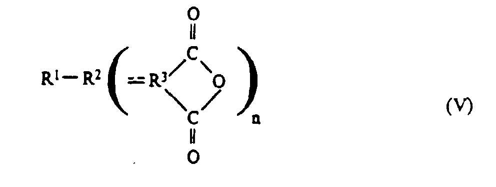 where
R¹, R² and R³ are as defined in one of claims 1 to 4, and
n is 1 or 2;
or
a compound of the formula VI 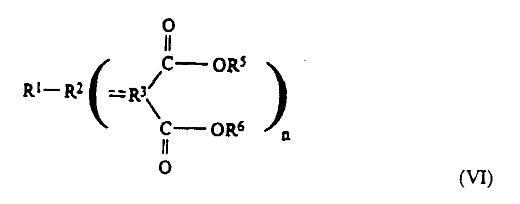 where
R¹, R², R³ and n are as defined above, and
R⁵ and R⁶ in each case independently of one another are a hydrogen atom or
an alkyl radical, in particular a C₁-C₄-alkyl radical;
being reacted with a monomeric polyalcohol or an oligomeric polyalcohol of the formula VII
A(⊙H)ₐ (VII)
where A, ⊙ and a are as defined in one of claims 1 to 4, in the presence of at least one catalyst or of a mixture of a number of catalysts, the catalyst or the catalysts preferably being a weakly acidic catalyst, a Lewis acid catalyst and/or a basic catalyst.

## Revendications

1. Composé de formule I
(H⊙)_{a-b}A(⊙X)_{b} (I)
dans laquelle
A représente le reste d'un polyalcool monomère ou d'un polyalcool oligomère, constitué de jusqu'à 20 monomères reliés entre eux par des liaisons éther, où le polyalcool monomère, respectivement le monomère du polyalcool oligomère est un alcool qui présente au moins 3 atomes de carbone ainsi que a groupements OH ;
a est ≥ 2;
Θ représente à chaque fois un atome d'oxygène d'un des groupements OH du reste A ;
X représente un reste de formule II 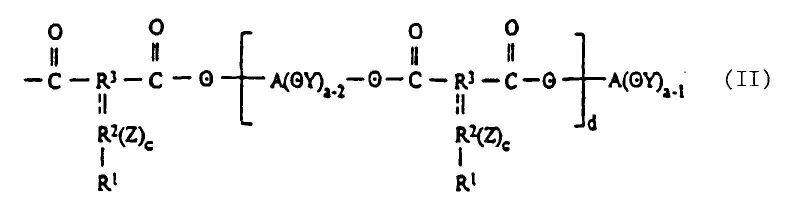
où
A, Θ et a sont tels que définis ci-dessus ;
Y représente un atome d'hydrogène ou un reste X tel que défini ci-dessus;
R¹ représente un reste alcényle ou alkyle à chaîne droite ou ramifiée présentant au moins 8 atomes de carbone ;
R² représente une liaison carbone-carbone ou un reste alcénylène ou alkylène à chaîne droite ou ramifiée ;
où R¹ et R² présentent ensemble jusqu'à 30 atomes de carbone, de préférence jusqu'à 20 atomes de carbone, et de préférence 0 à 3 doubles liaisons ;
R³ représente un reste méthylène, éthylène ou n-propylène, de préférence un reste éthylène ;
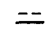 représente une liaison simple ou double ;
Z représente un reste de formule III 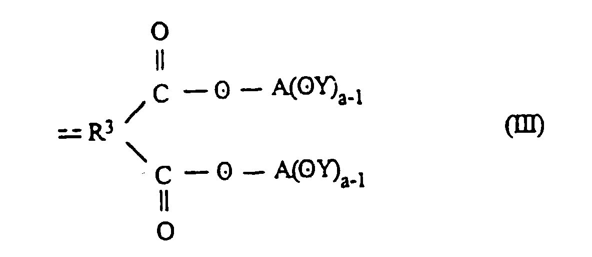
où
A, Θ, a, Y et 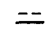 sont tels que définis ci-dessus ;
c vaut 0 ou 1;
d représente un nombre entier ≥ 1, de préférence de 1 à 100, de façon particulièrement préférée de 1 à 20, de façon plus particulièrement préférée de 1 à 4 ;
et
b représente un nombre entier qui s'élève au moins à 1 et au plus à a, qui est tel que défini ci-dessus ;
et dont le poids moléculaire (moyenne en poids) est de préférence ≤ 100 000 g/mol, de préférence ≤ 50 000 g/mol et de façon particulièrement préférée ≤ 20 000 g/mol.

2. Composé de formule I selon la revendication 1, où R¹ et R² représentent ensemble un reste alkyle ou alcényle à chaîne linéaire comportant en tout 8 à 20 atomes de carbone, et lorsque 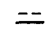 est mis pour une double liaison, R¹ et R² ne présentent pas d'autres doubles liaisons, et lorsque 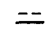 est mis pour une simple liaison, R¹ et R² présentent en tout une double liaison.

3. Composé selon l'une des revendications 1 ou 2 , caractérisé par la formule IV 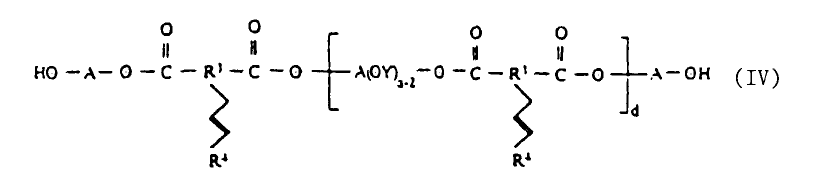 où
A, Θ, R³, Y et d sont tels que définis dans la revendication 1, et
R⁴ représente un reste alkyle à chaîne droite ayant 5 à 27, de préférence 5 à 17 atomes de carbone, de façon particulièrement préférée un reste n-pentyle, n-heptyle, n-nonyle ou n-undécyle.

4. Composé selon l'une quelconque des revendications 1 à 3,
où
le reste A est choisi parmi le glycérol, le glycérol oligomère ayant jusqu'à 20 motifs glycérol, le pentaérythrol, le triméthylolpropane et les sucres-alcools et les anhydrosucres-alcools, comme en particulier le sorbitol, le mannitol, l'adonitol, l'arabitol, le xylitol, le dulcitol, le sorbide, le sorbitanne et l'érythrol ;
de préférence parmi le glycérol et le glycérol oligomère ayant jusqu'à 20 motifs glycérol ;
de façon particulièrement préférée parmi le glycérol oligomère ayant de 2 à 10 motifs glycérol.

5. Utilisation d'un composé tel que défini dans l'une des revendications 1 à 4, en tant que stabilisant, qu'émulsifiant et/ou que substance active de lavage.

6. Utilisation selon la revendication 5, dans des compositions cosmétiques, des agents de lavage et de nettoyage, dans des compositions pharmaceutiques, de la nourriture diététique ou non diététique ou dans des agents phytosanitaires.

7. Composition cosmétique comprenant au moins une substance insoluble ou difficilement soluble dans l'eau et éventuellement des adjuvants et/ou des additifs usuels, caractérisée en ce que la composition cosmétique comprend au moins un composé de formule I selon l'une quelconque des revendications 1 à 4, en particulier en tant que solubilisant et/ou émulsifiant.

8. Agent de lavage et/ou de nettoyage, en particulier pour le nettoyage de surfaces, de textiles et/ou du corps, comprenant éventuellement des substances usuelles actives de lavage ainsi que des adjuvants et/ou des additifs usuels, caractérisé en ce que l'agent de lavage ou de nettoyage comprend au moins un composé de formule I selon l'une quelconque des revendications 1 à 4, en particulier en tant que substance active de lavage.

9. Composition pharmaceutique comprenant au moins une substance active, un adjuvant ou un additif insoluble ou difficilement soluble dans l'eau et éventuellement d'autres substances actives, adjuvants ou additifs, caractérisée en ce que la composition pharmaceutique comprend au moins un composé de formule I selon l'une quelconque des revendications 1 à 4, en particulier en tant que solubilisant et/ou émulsifiant.

10. Nourriture diététique ou non diététique, comprenant au moins une substance alimentaire, un adjuvant ou un additif insoluble ou difficilement soluble dans l'eau, caractérisée en ce que la nourriture comprend au moins un composé de formule I selon l'une quelconque des revendications 1 à 4, en particulier en tant que solubilisant et/ou émulsifiant.

11. Agent phytosanitaire, comprenant au moins une substance active, un adjuvant ou un additif insoluble ou difficilement soluble dans l'eau, caractérisé en ce que l'agent phytosanitaire comprend au moins un composé de formule I selon l'une quelconque des revendications 1 à 4, en particulier en tant que solubilisant et/ou émulsifiant.

12. Procédé de préparation d'un composé de formule I selon l'une quelconque des revendications 1 à 4, caractérisé en ce que l'on fait réagir un composé de formule V 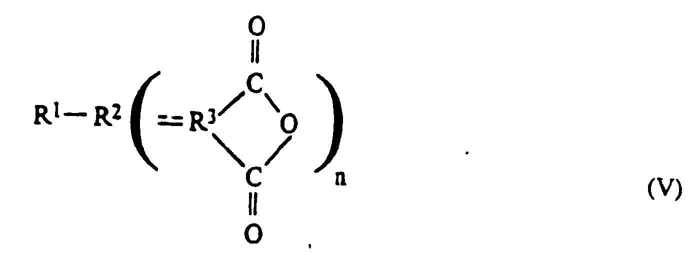 où
R¹, R² et R³ sont définis tels que dans l'une des revendications 1 à 4 et
n est mis pour 1 ou 2;
ou
un composé de formule VI 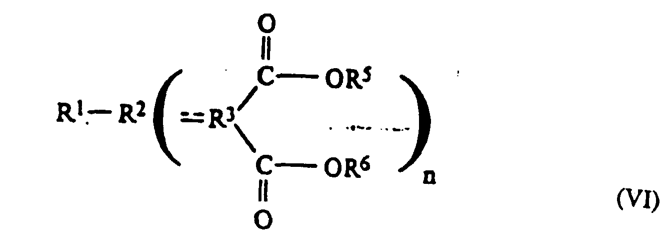 où
R¹, R² et R³ et n sont tels que définis ci-dessus, et
R⁵ et R⁶ représentent chacun indépendamment l'un de l'autre un atome d'hydrogène ou
un reste alkyle , en particulier un reste alkyle en C₁-C₄ ;
avec un polyalcool monomère ou un polyalcool oligomère de formule VII
A(⊙H)ₐ (VII)
où A, Θ et a sont tels que définis dans une des revendications 1 à 4, en présence d'au moins un catalyseur ou d'un mélange de plusieurs catalyseurs, où le catalyseur ou les catalyseurs sont de préférence un catalyseur acide fort, un catalyseur acide de Lewis et/ou un catalyseur basique.
